(19) Europäisches Patentamt
European Patent Office
Office européen des brevets

(11) EP 0 956 866 A1

(12) **EUROPEAN PATENT APPLICATION**
published in accordance with Art. 158(3) EPC

(43) Date of publication:
**17.11.1999 Bulletin 1999/46**

(21) Application number: **97949125.5**

(22) Date of filing: **17.12.1997**

(51) Int. Cl.$^6$: **A61K 45/00**, A61K 31/47, A61K 31/44, A61K 31/50, A61K 31/38, A61K 31/275

(86) International application number:
**PCT/JP97/04665**

(87) International publication number:
**WO 98/29135 (09.07.1998 Gazette 1998/27)**

(84) Designated Contracting States:
**AT BE CH DE DK ES FI FR GB GR IE IT LI LU MC NL PT SE**

(30) Priority: **26.12.1996 JP 34873896**

(71) Applicant:
**KIRIN BEER KABUSHIKI KAISHA**
**Chuo-Ku, Tokyo 104 (JP)**

(72) Inventors:
- **OGAWA, Takahiro**
  **Nishinomiya-shi, Hyogo 662 (JP)**
- **WATANABE, Noriko**
  **Suita-shi, Osaka 564 (JP)**
- **WAKI, Mitsunori**
  **Kobe-shi, Hyogo 651-22 (JP)**

(74) Representative: **HOFFMANN - EITLE**
**Patent- und Rechtsanwälte**
**Arabellastrasse 4**
**81925 München (DE)**

(54) **DRUGS FOR AMELIORATING OPHTHALMIC CIRCULATORY DISTURBANCE**

(57) Drugs for ameliorating ophthalmic circulatory disturbance characterized by containing as the active ingredient potassium channel openers. As the potassium channel openers, use may be preferably mado of carboxyimidamide derivatives represented by general formula (I), acid-addition salts thereof or hydrates of the same, wherein each symbol is as defined in the specification. When dropped into eyes, in particular, drugs for ameliorating ophthalmic circulatory disturbance containing the compounds of general formula (I) can increase the blood flow in the optic papillae of normal eyes and inhibit retinal vessel contraction and a decrease in the blood flow in the optic papillae of eyes suffering from circulatory disturbance caused by ET-1 while scarcely affecting the blood pressure. It is therefore suggested that these compounds are useful as remedies for glaucoma, in particular, normotensive glaucoma caused by ophthalmic circulatory disturbance, retinal pigment degeneration, macular degeneration, ischemic optic nerve disease, iridocyclitis, retinal arterial obliteration, retinal phlebemphraxis, diabetic retinopathy, ischemic optic nerve disease, choroid diseases following retinal lesions, retinal choroid diseases accompanied by systemic diseases, etc.

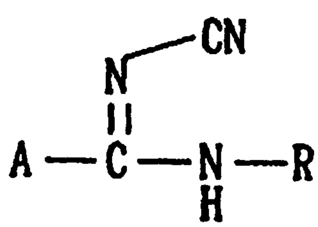

(I)

EP 0 956 866 A1

## Description

## Technical Field

The present invention relates to an agent for ameliorating ocular circulatory disorder, which contains a potassium channel opener. More particularly, the present invention relates to an agent for ameliorating ocular circulatory disorder, which contains a specific carboxyimidamide derivative, an acid addition salt thereofor a hydrate thereof as an active ingredient.

## Background Art

An intraocular blood circulation (hereinafter to be referred to as ocular circulation) has two major pathways, one being a circulation via ciliary artery and the other being a circulation via central retinal artery. The ciliary artery is connected to the arteries of choroid, optic nerve head, iris, ciliary body and the like, and the blood is discharged from the eye through the vortex vein. The central retinal artery passes through optic nerve and is connected to the central retinal vein, wherein apart thereof is branched into arteriola at the optic nerve head and then into capillary. Of these, the circulatory disorder of ciliary artery includes glaucoma (particularly normal tension glaucoma), retinitis pigmentosa, macular degeneration, ischemic optic neuropathy, iridocylitis and the like. The circulatory disorder of the central retinal artery includes retinal artery occlusion, retinal vein occlusion, diabetic retinopathy, ischemic optic neuropathy, choroidal disease following retinal lesion, retinochoroidal disease associated with systemic disease and the like. As is evident from the description of the above-mentioned ocular circulation pathway, the diseases caused by the above-mentioned ocular circulation disorders make an onset when the smooth circulation of retina, optic nerve head, choroid, iris, ciliary body and the like is prevented.

In recent years, there has been a high incidence of glaucoma, in particular, normal tension glaucoma (also referred to as low tension glaucoma) due to blood circulatory disorder in optic nerve head, and therapeutic method of the disease has been desired. By the epidemiological studies in recent years, normal tension glaucoma has been elucidated to be of a disease type of glaucoma having the highest incidence, though the intraocular pressure is in the normal range, so that it is considered to be distinct from the generally known glaucoma, namely the glaucoma caused by a high intraocular pressure. The normal tension glaucoma is generally considered to be a disease associated with (1) an intraocular pressure including a biological rhythm of not more than 21 mmHg, (2) a normal open angle, (3) glaucomatous excavation of optic nerve head and the corresponding visual field defect, (4) no intracranial lesion or paranasal sinuses disease which can cause optic atrophy and (5) no mass hemorrhage or shock [Low Tension Glaucoma and endotheline (ET-1), *Folia Ophthalmologica Japonica*, vol. 43, pp.554-559 (1992) and Low Tension Glaucoma- History and Concept, *Journal of the Eye*, vol. 8, pp. 493-500 (1991)).

A recent report has documented that an oral administration of a circulation ameliorating agent to a patient with normal tension glaucoma resulted in an increased optic nerve head blood flow and an ameliorated normal tension glaucoma [Influence of $Ca^2$ antagonist on changes in visual field in low tension glaucoma, Journal of *Japanese Ophthalmological Society*, vol. 92, pp. 792-797 (1988)]. There is a possibility that various biological vasocontracting substances that decrease blood flow may be involved in patients with normal tension glaucoma, and a significantly higher endotheline-1 (hereinafter sometimes referred to as ET-1) concentration in blood as compared to the level in healthy subjects has been reported [(Low Tension Glaucoma and Endotheline (ET-1), *Folia Ophthalmolgica Japonica*, vol. 43, pp. 554-559 (1992)]. ET-1 is considered to act on a receptor present in vascular smooth muscle cells and directly opens the voltage-dependent calcium channel, as well as releases calcium ion from intracellular sarcoplasmic reticulum [Intracellular Signal Transduction Pathway Relating to the Action and Regulation of Release of Endotheline, Experimental Medicine, vol. 8, pp. 28-35 (1990)].

Moreover, a decrease in optic nerve head blood flow by an injection of ET-1 into the vitreous body has been disclosed [Effect of Endotheline-1 on Ocular Circulation, *Journal of Japanese Ophthalmological Society*, vol. 97, pp. 678-682 (1993)]. From these, a possible involvement of various biological vasocontracting substances is suggested with respect to circulatory disorders in normal tension glaucoma, of which ET-1 is particularly plausible, wherein the possibility is suggested that ET-1 may cause an ocular circulation disorder and decrease optic nerve head blood flow. Therefore, amelioration of an ocular circulation disorder induced by ET-1 is considered to be one of the effective therapeutic methods of normal tension glaucoma.

The ocular circulation disorder is most frequently seen among the retinochoroidal diseases. The retinochoroidal disease caused by an ocular circulation disorder is exemplified by retinal artery occlusion, retinal vein occlusion, diabetic retinopathy, retinitis pigmentosa, macular degeneration, choroidal disease following retinal lesion, retinochoroidal disease accompanied by systemic disease, and the like. While the etiology of retinal artery occlusion and retinal vein occlusion is unknown, the lumen of retinal artery or vein is occluded to cause circulation disorder in retina and optic nerve head. In addition, the high ET-1 concentration in blood of a patient has been also reported [Deviation of Vasospasm Factor in Retinal Artery Occlusion, *Japanese Journal of Clinical Ophthalmology*, vol. 46, pp. 431-434 (1992)]. It is a well-known fact that thrombosis occurs in retinal blood vessel in diabetic retinopathy, which in turn causes retinal circulatory disorder. The retinitis pigmentosa is a binocular retinal disease, which starts with night blindness in school age, gradually progresses into abnormal visual field and visual loss, and may ultimately end in blindness. This disease is hereditary and the degeneration of retinal photoreceptor cell proceeds with increasingly narrower retino choroidal blood vessel and circulatory disorders. An ocular circulation disorder is said to be observed in macular degeneration as well. The above-mentioned diseases that accompany ocular circulation disorder have been treated by an oral administration of tocopherol nicotinate (Juvela N : vitamin E preparation manufactured by EISAI CO., LTD.)

The optic nerve disease associated with an optic nerve disorder is exemplified by ischemic optic neuropathy and the like. The ischemic optic neuropathy gives an onset by circulatory disorder of optic nerve nutrient blood vessel. The disease accompanied by iris ciliary body circulatory disorder is exemplified by iridocyclitis and the like.

A potassium channel opener is known as a pharmaceutical agent having peripheral vasodilating action, which opens the potassium channel, hyperpolarizes cell membrane, and prevents intracellular influx of calcium ion necessary for the contraction of cardiac muscle and vascular smooth muscle and prevents release of calcium ion from intracellular sarcoplasmic reticulum, whereby to relax the vascular smooth muscle to dilate blood vessel, thus increasing blood flow.

WO89/ 10757 discloses that potassium channel openers such as pinacidil, cromakalim and the like lower the ocular pressure, but does not teach that they increase the ocular blood flow.

A publication (Journal of Ocular Pharmacology and Therapeutics, 1995, 11(3), 195-201) discloses that cromakalim lowers the ocular blood flow, and therefore, is inappropriate for the patients with an eye disease.

The present applicant has already disclosed that certain carboxyimidamide derivative has a potassium channel opening action, such as vasodilating action and the like, and is useful as antihypertensive agent, coronaxy vasodilator and the like in Japanese Patent Examined Publication No. 6-62567 (Japanese Patent Unexamined Publication No. 3-163061), Japanese Patent Unexamined Publication No. 3-218343, EP 388,528 and WO93/15057.

However, the above-mentioned publications fail to disclose or suggest that the vasodilating action is expressed on retinochoroid. They also fail to referto the inhibition of contraction of blood vessel by ET-1. Moreover, these references do not disclose that this derivative is useful for the prophylaxis and treatment of glaucoma (particularly normal tension glaucoma caused by ocular circulation disorder), retinitis pigmentosa, macular degeneration, isehemic optic neuropathy, iridocyclitis, retinal artexy occlusion, retinal vein occlusion, diabetic retinopathy, choroidal disease following retinal lesion, retinochoroidal disease accompanied by systemic disease and the like.

## Disclosure of the Invention

The present invention aims at solving the above-mentioned problems and has been made with the purpose of providing an agent for ameliorating an ocular circulatory disorder, which has a superior blood flow increasing action in retinochoroid and which has an inhibitory action on the vasocontraction and decrease in blood flow caused by ET-1 which is one of the biological vasocontracting substances.

The present inventors have conducted intensive studies in an attempt to achieve the above-mentioned objects and found that a potassium channel opener, particularly the above-mentioned carboxyimidamide derivative (also called carboxyamidine derivative, hereinafter to be referred to as carboxyimidamide derivative) shows a superior blood flow increasing action in retinochoroid and an inhibitory action on vasocontraction and on decrease in blood flow caused by ET-1, and that, particularly when in the form of an eye drop, the compound of the present invention scarcely has an influence on the blood pressure.

Accordingly, the present invention provides the following.

(1) An agent for ameliorating an ocular circulatory disorder, comprising a potassium channel opener except cromakalim as an active ingredient.

(2) The agent for ameliorating an ocular circulatory disorder of (1) above, wherein the potassium channel opener is acarboxyimidamide derivative of the following formula (I)

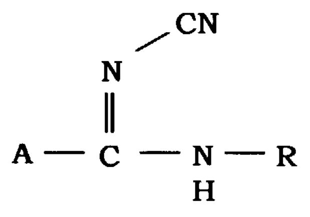

(I)

wherein A is a group of the formula

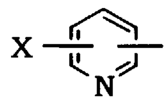

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom,

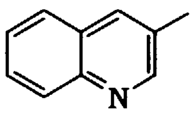 , 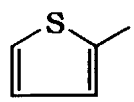 , 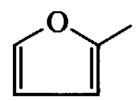 , 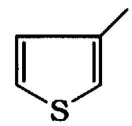 ,

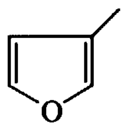 , 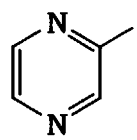 , 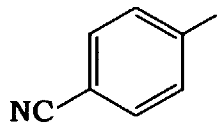

or $H_3C$-S-;
when A is

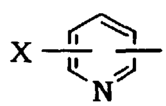

then R should be -$R^1$ a group of the formula

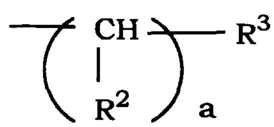

wherein $R^1$ is alkyl, alkyl having nitroxyl, a group of the formula

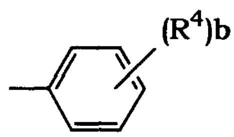

wherein $R^4$ is alkyl or alkoxyl, and b is 0 or 1, or

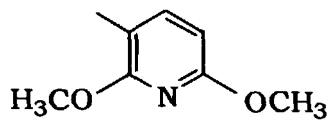

$R^2$ is one or more groups selected from the group consisting of alkyl, aryl, nitroxyl, arylalkoxyl, hydroxyl and hydrogen atom,
a is an integer of 1 to 3, and when a is 2 or above,two or more $R^2$ may be one or more from the groups and the atom of the above-mentioned group, and
$R^3$ is a group of the formula

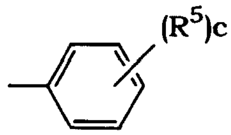

wherein $R^5$ is one or more groups selected from the group consisting of alkyl, alkoxyl, arylalkoxyl, nitro, amino, alkylamino, arylalkylamino, alkylthio, perfluoroalkyl and halogen atom, and c is an integer of 0 to 5, and when c is 2 or above, two or more $R^5$ may be one or more from the groups and the atom of the above-mentioned group,

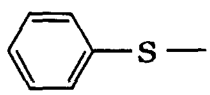 or 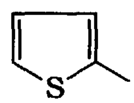

and when A is

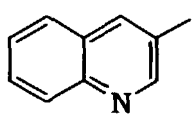 , 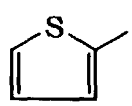 , 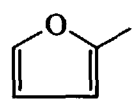 , 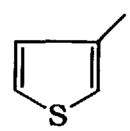 ,

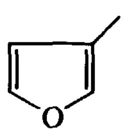 , 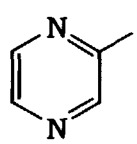 , 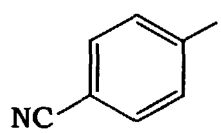

or $H_3C$-S-, then R should be

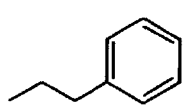 or 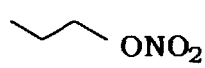

an acid addition salt thereofor a hydrate thereof.

(3) The agent for ameliorating an ocular circulatory disorder of (2) above, wherein the carboxyimidamide derivative of the formula (I) is a pyridine carboxyimidamide derivative of the following formula (II)

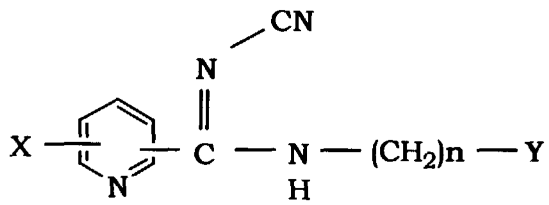

(II)

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom, Y is nitroxyl, methyl, or phenyl optionally substituted by alkyl, alkoxyl, arylalkoxyl, nitro, amino, alkylamino, arylalkylamino, alkylthio, perfluoroalkyl or halogen atom, and n is an integer of 0 to 3.

(4) The agent for ameliorating an ocular circulatory disorder of (3) above, wherein the pyridine carboxyimidamide derivative of the formula (II) is a pyridinecarboxyimidamide derivative of the following formula (III)

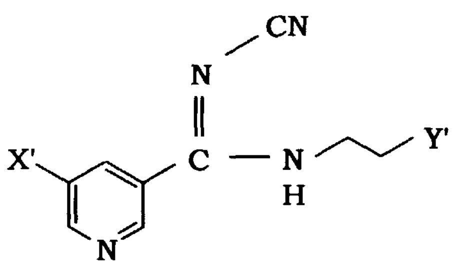

(III)

wherein X' is hydrogen atom or amino, and Y' is nitroxyl or 2-chlorophenyl

(5) The agent for ameliorating an ocular circulatory disorder of (4) above, wherein the pyridine carboxyimidamide derivative of the formula (III) is N-cyano-N'-(2-nitroxyethyl)-3-pyridinecarboxyimidamide.

(6) The agent for ameliorating an ocular circulatory disorder of (4) above, wherein the pyridinecarboxyimidamide derivative of the formula (III) is 5-amino-N-cyano-N'-[2-(2-chlorophenyl)ethyl]-3-pyridinecarboxyimidamide.

(7) The agent for ameliorating an ocular circulatory disorder of any of (1) to (6) above, which is in the form of an eye drop.

(8) The agent for ameliorating an ocular circulatory disorder of any of (1) to (6) above, which is in the form of an eye ointment.

(9) The agent for ameliorating an ocular circulatory disorder of any of (1) to (8) above, which is an agent for the prophylaxis and treatment of a disease caused by a circulatory disorder in the ciliary artery system.

(10) The agent for ameliorating an ocular circulatory disorder of (9) above, wherein the disease caused by the circulatory disorder in the ciliary artery system is a disease selected from the group consisting of glaucoma, retinitis pigmentosa, macular degeneration, ischemic optic neuropathy and iridocycitis.

(11) The agent for ameliorating an ocular circulatory disorder of (9) above, wherein the disease caused by the circulatory disorder in the ciliary artery system is normal tension glaucoma.

(12) The agent for ameliorating an ocular circulatory disorder of (4) above, which is an agent for the prophylaxis or treatment of glaucoma.

(13) The agent for ameliorating an ocular circulatory disorder of any of (1) to (8) above, which is an agent for the prophylaxis and treatment of a disease caused by a circulatory disorder in the central retinal artery system.

(14) The agent for ameliorating an ocular circulatory disorder of (13) above, wherein the disease caused by the circulatory disorder in the central retinal artery system is a disease selected from the group consisting of retinal artery occlusion, retinal vein occlusion, diabetic retinopathy, ischemic optic neuropathy, choroidal disease following retinal lesion and retinochoroidal disease accompanied by systemic disease.

## Brief Description of the Drawing

Fig. 1 is a graph showing the time course changes in relative optic nerve head blood flow (blood flow at each point in time when the initial value is 100%) in normal eye after instillation of a 0.001% ophthalmic solution (containing 0.001% of the compound a of the present invention) in Experimental Example 1, wherein the axis of abscissa shows time (mm) after instillation of the 0.001% ophthalmic solution and the axis of ordinate shows relative optic nerve head blood flow (%). Each value shows mean±standard error (n=6). A significant difference from the control is found in *1;P<0.05,*2;P<0.01,*3;P<0.001 (paired t-test). The black circle shows instillation of a 0.001% ophthalmic solution and white circle shows instillation of physiological saline.

Fig. 2 is a graph showing the time course changes in relative blood pressure (blood pressure at each time point when the initial value is 100%) of normal eye after instillation of a 0.001% ophthalmic solution (containing 0.001% inventive compound a) in Experimental Example 1, wherein the axis of abscissa shows time (mm) after instillation of the 0.001% ophthalmic solution and the axis of ordinate shows relative blood pressure (%). Each value shows mean ± standard error (n=5).

Fig. 3 is a graph showing the time course changes in relative optic nerve head blood flow (blood flow at each time point when the initial value is 100%) in normal eye after instillation of a $10^{-4}$% ophthalmic solution (containing $10^{-4}$% of the compound b of the present invention) in Experimental Example 2, the axis of abscissa shows time (min) after instillation of the $10^{-4}$% ophthalmic solution and the axis of ordinate shows relative optic nerve head blood flow (%). Each value shows mean±standard error (n=6). A significant difference from the control is found in *;P<0.05,**;P<0.01; ***;P<0.001 (paired t-test). The black circle shows instillation of the $10^{-4}$% ophthalmic solution and white circle shows instillation of physiological saline.

Fig. 4 is a graph showing the time course changes in relative optic nerve head blood flow upon injection of ET-1 after instillation of a $10^{-4}$% ophthalmic solution (containing $10^{-4}$% of the compound b of the present invention) in Experimental Example 3, wherein the axis of abscissa shows time (mm) when the injection of ET-1 is 0 and the axis of ordinate shows relative optic nerve head blood flow (%). Each value shows mean±standard error (n=6). A significant difference from the control is found in *;P<0.01,**;P<0.001 (paired t-test) in the variation in the blood flow at each point in time from that immediately before the injection of ET-1. The black circle shows instillation of the $10^{-4}$% ophthalmic solution and white circle shows instillation of physiological saline.

Fig. 5 is a graph showing the time course changes in retinal vasocontraction upon injection of ET-1 after instillation of a $10^{-4}$% ophthalmic solution (containing $10^{-4}$% of the compound b of the present invention) in Experimental Example 3, wherein the axis of abscissa shows time (mm) when the injection of ET-1 is 0 and the axis of ordinate shows score of the retinal blood vessel diameter. Each value shows mean±standard error (n=7). A significant difference from the control is found in *;P<0.05 (Wilcoxon test). The black circle shows instillation of the $10^{-4}$% ophthalmic solution and white circle shows instillation of physiological saline.

## Detailed Description of the Invention

The present invention is described in detail in the following. The potassium channel opener to be used in the present invention is exemplified by benzopyran derivatives such as lemakalim and the like, guanidine derivatives such as pinacidil and the like, pyridine derivatives such as nicorandil and the like, pyrimidine derivatives such as minoxidil and the like, benzothiazide derivatives such as chlorothiazide and the like, and the like. In the present invention, carboxyimidamide derivative of the following formula (I), an acid addition salt thereof and a hydrate thereof are preferably used.

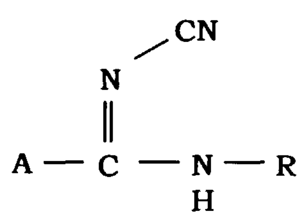

(I)

wherein A is a group of the formula

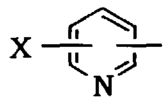

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom,

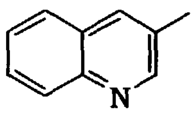 , 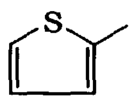 , 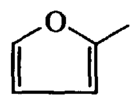 , 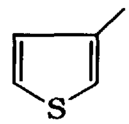 ,

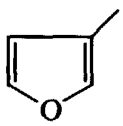 , 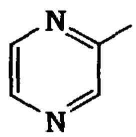 , 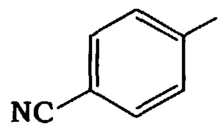

or $H_3C$-S-;
when A is

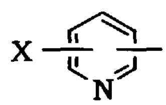

then R should be -$R^1$ a group of the formula

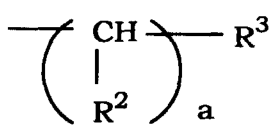

wherein $R^1$ is alkyl, alkyl having nitroxyl, a group of the formula

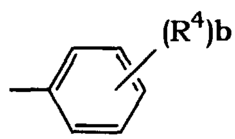

wherein $R^4$ is alkyl or alkoxyl, and b is 0 or 1, or

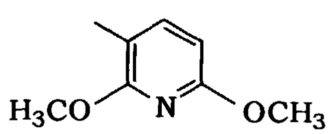

$R^2$ is one or more groups selected from the group consisting of alkyl, aryl, nitroxyl, arylalkoxyl, hydroxyl and hydrogen atom,

a is an integer of 1 to 3, when a is 2 or above, two or more $R^2$ may be one or more from the groups and the atom of the above-mentioned group, and

$R^3$ is a group of the formula

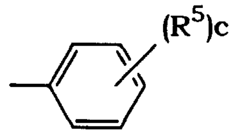

wherein $R^5$ is one or more groups selected from the group consisting of alkyl, alkoxyl, arylalkoxyl, nitro, amino, alkylamino, arylalkylamino, alkylthio, perfluoroalkyl and halogen atom, and c is an integer of 0 to 5, and when c is 2 or above, two or more $R^5$ may be one or more from the groups and the atom of the above-mentioned group,

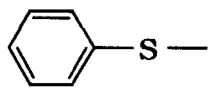 or 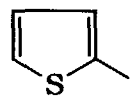

and when A is

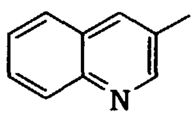, 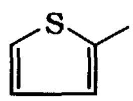, 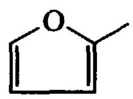, 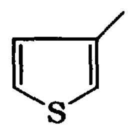,

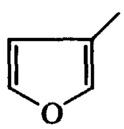, 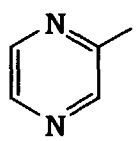, 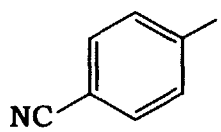

or $H_3C$-S-, then R should be

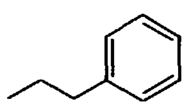 or 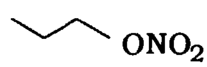

an acid addition salt thereofor a hydrate thereof.

In the formula (I), alkyl at X may be linear or branched and preferably has 1 to 5, particularly 1 to 3, carbon atoms. Examples thereof include methyl, ethyl, n-propyl, isopropyl and the like. The hydroxyalkyl at X may be linear or branched and preferably has 1 to 5, particularly 1 to 3, carbon atoms. Examples thereof include hydroxymethyl, 2-hydroxyethyl, 2-hydroxypropyl, 3-hydroxypropyl and the like. The acylamino at X preferably has 1 to 10, particularly 2 to 7, carbon atoms. Examples thereof include acetylamino, propionylamino, butyrylamino, benzoylamino and the like. The alkylamino at X may be linear or branched and preferably has 1 to 5, particularly 1 to 3, carbon atoms. Examples thereof include methylamino, ethylamino, propylamino, isopropylamino and the like. The dialkylamino at X may be linear or branched and preferably has 1 to 5, particularly 1 to 3, carbon atoms. Examples thereof include dimethylamino, diethylamino, dipropylamino, diisopropylamino and the like. The aryl moiety of arylalkylamino at X maybe unsubstituted or substituted and the alkylene moiety may be linear or branched, but preferably has 1 to 5, particularly 1 to 3, carbon

atoms. Examples of arylalkylamino include benzylamino, phenethylamino, 3-phenylpropylamino and the like. The alkylsulfonamido at X may be linear or branched and preferably has 1 to 5, particularly 1 to 3, carbon atoms. Examples thereof include methanesulfonamido, ethanesulfonamido, propanesulfonamido and the like. The bisalkylsulfonylamino at X may be linear or branched and preferably has 1 to 5, particularly 1 to 3, carbon atoms. Examples thereof include bismethanesulfonylamino and the like. Halogen at X is fluorine atom, chlorine atom, bromine atom or iodine atom, with preference given to fluorine atom, chlorine atom and bromine atom.

In the formula (I), alkyl at $R^1$ may be linear or branched and preferably has 1 to 10, particularly 5 to 8, carbon atoms. Examples thereof include 2,2-dimethylpropyl, 1,2,2-trimethylpropyl, 3,3-dimethylbutyl, 4,4-dimethylpentyl, 5,5-dimethylhexyl, 6-methylheptyl, octyl and the like. The alkyl having nitroxylat $R^1$ preferably has 2 to 5, particularly 2 to 4, carbon atoms. In this case, plural nitroxyl may be included, with particular preference given to one or two nitroxyl. The nitroxyl may be bonded to any of primary to tertiary carbons, particularly primary carbon. Examples of such alkyl having nitroxyl include 2-nitroxyethyl, 3-nitroxypropyl, 1-methyl-2-nitroxyethyl, 4-nitroxybutyl, 1-methyl-3-nitroxypropyl, 2-methyl-3-nitroxypropyl, 1,1-dimethyl-2-nitroxyethyl and the like.

In the formula (I), alkyl at $R^2$ preferably has 1 to 5, particularly 1 to 3, carbon atoms. Examples thereof include methyl, ethyl, n-propyl, isopropyl and the like. The aryl at $R^2$ may be unsubstituted or substituted. Preferable examples thereof include tolyl, xylyl and phenyl, with more preference given to phenyl. The aryl moiety of arylalkoxyl at $R^2$ may be unsubstituted or substituted and the alkoxyl moiety preferably has 1 to 5, particularly 1 to 3, carbon atoms. Examples of such arylalkoxyl include phenethyloxyl, 3-phenylpropyloxyl and benzyloxyl, with more preference given to benzyloxyl. When $R^2$ is included in plurality at the same time, they may be one or more from the above-mentioned groups and the atom.

In the formula (I), alkyl at $R^4$ preferably has 1 to 5, particularly 1 to 3, carbon atoms. Examples thereof include methyl, ethyl, n-propyl, isopropyl and the like. The alkoxylat $R^4$ preferably has 1 to 5, particularly 1 to 3, carbon atoms. Examples thereof include methoxy, ethoxy, n-propoxy, isopropoxy and the like.

In the formula (I), alkyl at $R^5$ preferably has 1 to 5, particularly 1 to 3, carbon atoms. Examples thereof include methyl, ethyl, n-propyl, isopropyl and the like. The alkoxyl at $R^5$ preferably has 1 to 5, particularly 1 to 3, carbon atoms. Examples thereof include methoxy, ethoxy, n-propoxy, isopropoxy and the like. The aryl moiety of arylalkoxyl at $R^5$ may be unsubstituted or substituted and the alkoxyl moiety preferably has 1 to 5, particularly 1 to 3, carbon atoms. Examples of such arylalkoxyl include phenethyloxyl, 3-phenylpropyloxyl and benzyloxyl, with preference given to benzyloxyl. Alkylamino at $R^5$ preferably has 1 to 5, particularly 1 to 3, carbon atoms. Examples thereof include methylamino, ethylamino, n-propylamino, isopropylamino and the like. The aryl moiety of arylalkylamino at $R^5$ maybe unsubstituted or substituted and the alkylene moiety preferably has 1 to 5, particularly 1 to 3, carbon atoms. Examples of such arylalkylamino include benzylamino, phenethylamino, 3-phenylpropylamino and the like. The alkylthio at $R^5$ preferably has 1 to 5, particularly 1 to 3, carbon atoms. Examples thereof include methylthio, ethylthio, n-propylthio, isopropylthio and the like. The perfluoroalkyl at $R^5$ preferably has 1 to 5, particularly 1 to 3, carbon atoms. Examples thereof include trifluoromethyl. The halogen atom at $R^5$ is fluorine atom, chlorine atom, bromine atom or iodine atom, with preference given to fluorine atom, chlorine atom and bromine atom. $R^5$ means having one of these groups and atoms, as well as having plural kinds thereof.

The carboxyimidamide derivative (I) of the present invention has basic nitrogen atom and is capable of forming an acid addition salt. The acid used to form an acid addition salt is subject to no particular limitation and may be any as long as it is pharmacologically acceptable and nontoxic. Examples thereof include inorganic acid (e.g., hydrochloric acid, sulfuric acid, nitric acid and phosphoric acid), organic acid (e.g., acetic acid, propionic acid, maleic acid, oleic acid, palmitic acid, citric acid, succinic acid, tartaric acid, fumaric acid, glutamic acid, pantothenic acid and methanesulfonic acid) and the like. The carboxyimidamide derivative (I) of the present invention and an acid addition salt thereof may be hydrates.

The potassium channel opener, particularly carboxyimidamide derivative (I), has a blood flow increasing action in retinochoroid and inhibitory action on vasocontraction and on decrease in blood flow caused by ET-1, which is one of the biological vasocontracting substances, in mammals (e.g., human, cow, horse, mouse, rat, dog, cat, rabbit and the like). When particularly in the form of an eye drop, it scarcely effects the blood pressure.

With regard to the agent for ameliorating an ocular circulatory disorder of the present invention, the pyridinecarboxyimidamide derivative of the following formula (II), from among the carboxyimidamide derivatives of the formula (I), shows particularly superior action of the present invention:

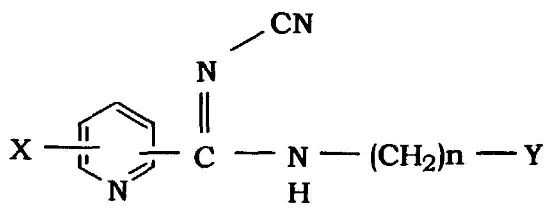

(II)

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom, Y is nitroxyl, methyl, or phenyl optionally substituted by alkyl, alkoxyl, arylalkoxyl, nitro, amino, alkylamino, arylalkylamino, alkylthio, perfluoroalkyl or halogen atom, and n is an integer of 0 to 3.

With regard to the agent for ameliorating an ocular circulatory disorder of the present invention, the pyridinecarboxyimidamide derivative of the following formula (III), from among the pyridinecarboxyimidamide derivatives of the formula (II), shows the most superior action of the present invention:

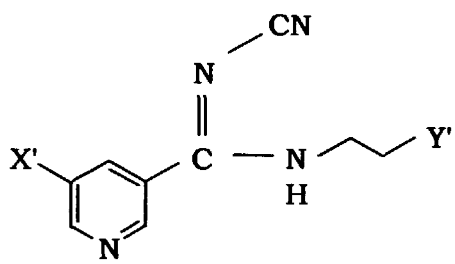

(III)

wherein X' is hydrogen atom or amino and Y' is nitroxyl or 2-chiorophenyl.

Preferable examples of such carboxyimidamide derivative (III) of the present invention are, for example, the following compounds.

- N-cyano-N'-(2-nitroxyethyl)-3-pyridinecarboxyimidamide (also called N-cyano-N'-(2-nitroxyethyl)-3-pyridinecarboxyamidine, hereinafter sometimes referred to as the compound a of the present invention)
- 5-amino-N-cyano-N'-[2-(2-chlorophenyl)ethyl]-3-pyridinecarboxyimidamide (5-amino-N-[2-(2-chlorophenyl)ethyl]-N'-cyano-3-pyridinecarboxyamidine, hereinafter sometimes referred to as the compound b of the present invention)

Therefore, a potassium channel opener, particularly carboxyimidamide derivative (I), an acid addition salt thereof and a hydrate thereof (hereinafter sometimes referred to as the compound of the present invention) are useful as an agent for ameliorating an ocular circulatory disorder in mammals such as human, cow, horse, dog, mouse, rat and the like, and they are expected to be applicable for the prophylaxis and treatment of glaucoma (particularly normal tension glaucoma caused by an ocular circulation disorder), retinitis pigmentosa, macular degeneration, ischemic optic neuropathy, iridocycitis, retinal artery occlusion, retinal vein occlusion, diabetic retinopathy, choroidal disease following retinal lesion, retinochoroidal disease accompanied by systemic disease and the like.

When the inventive compound is used as a pharmaceutical product, it can be administered orally or parenterally. The administration route includes oral administration in tablet, capsule, syrup and the like, and parenteral administration of a liquid injection of solution, emulsion, suspension and the like, parenteral administration of an external preparation such as ointment (particularly eye ointment), cream, suppository, poultice, eye drop, nasal drop, inhalent, liniment, aerosol and the like. In consideration of influence on other circulatory system, the particularly preferable dosage form is an eye drop and an eye ointment.

The preparation in the above-mentioned dosage form can be produced according to the conventional method comprising adding a typical additive necessary for producing preparations, such as carrier, excipient, binder, stabilizer and the like, to the compound of the present invention and formulating the same.

While the dose and administration frequency of the agent for ameliorating an ocular circulatory disorder of the

present invention vary depending on symptom, age, bodyweight and administration route, when, for example, administered as an eye drop to an adult, a preparation containing the inventive compound at a concentration of 0.0000001-0.5w/v%, preferably 0.000001-0.1 w/v%, can be administered several times, preferably 1 to 6 times, a day by several drops, preferably 1 to 3 drops, each time. When in use as an eye ointment, a preparation containing the inventive compound at a concentration of 0.0000001-0.5 w/v%, preferably 0.000001-0.1 w/v%, can be applied several times, preferably 1 to 6 times, a day.

The present invention is explained in more detail in the following by way of Examples and Experimental Examples.

## Example 1

An aqueous ophthalmic solution containing the compound of the present invention was prepared according to the following prescription.

| | |
|---|---|
| Compound a of the present invention | 0.001 g |
| Sodium dihydrogenphosphate | 0.1 g |
| Sodium chloride | 0.85g |
| Benzalkonium chloride | 0.005g |
| Sterile purified water | suitable amount |
| Total amount | 100 ml (pH 7) |

## Example 2

An aqueous ophthalmic solution containing the compound of the present invention was prepared according to the following prescription.

| | |
|---|---|
| Compound b of the present invention | 0.001 g |
| Sodium dihydrogenphosphate | 0.1 g |
| Sodium chloride | 0.85g |
| Benzalkonium chloride | 0.005 g |
| Sterile purified water | suitable amount |
| Total amount | 100 ml (pH 7) |

## Example 3

An aqueous ophthalmic solution containing the compound of the present invention was prepared according to the following prescription.

| | |
|---|---|
| Compound a of the present invention | 0.001 g |
| Sodium dihydrogenphosphate | 0.1 g |
| Glycerine | 2.5g |
| Benzalkonium chloride | 0.005g |
| Sterile purified water | suitable amount |

(continued)

| | |
|---|---|
| Total amount | 100 ml (pH 7) |

**Example 4**

An aqueous ophthalmic solution containing the compound of the present invention was prepared according to the following prescription.

| | |
|---|---|
| Compound b of the present invention | 0.001 g |
| Sodium dihydrogenphosphate | 0.1 g |
| Glycerine | 2.5g |
| Benzalkonium chloride | 0.005g |
| Sterile purified water | suitable amount |
| Total amount | 100 ml (pH 7) |

**Example 5**

An aqueous ophthalmic solution containing the compound of the present invention was prepared according to the following prescription.

| | |
|---|---|
| Compound a of the present invention | 0.001 g |
| Sodium acetate | 0.1 g |
| Sodium chloride | 0.85 g |
| Benzalkonium chloride | 0.005 g |
| Sterile purified water | suitable amount |
| Total amount | 100 ml (pH 5) |

**Example 6**

An aqueous ophthalmic solution containing the compound of the present invention was prepared according to the following prescription.

| | |
|---|---|
| Compound b of the present invention | 0.001 g |
| Sodium acetate | 0.1 g |
| Sodium chloride | 0.85g |
| Benzalkonium chloride | 0.005 g |
| Sterile purified water | suitable amount |
| Total amount | 100 ml (pH 5) |

**Example 7**

An aqueous ophthalmic solution containing the compound of the present invention was prepared according to

the following prescription.

| | |
|---|---|
| Compound <u>a</u> of the present invention | 0.001 g |
| Sodium acetate | 0.1 g |
| Glycerine | 2.5g |
| Benzalkonium chloride | 0.005g |
| Sterile purified water | suitable amount |
| Total amount | 100 ml (pH 5) |

**Example 8**

An aqueous ophthalmic solution containing the compound of the present invention was prepared according to the following prescription.

| | |
|---|---|
| Compound <u>b</u> of the present invention | 0.001 g |
| Sodium acetate | 0.1 g |
| Glycerine | 2.5g |
| Benzalkonium chloride | 0.005g |
| Sterile purified water | suitable amount |
| Total amount | 100 ml (pH 5) |

**Example 9**

An eye ointment containing the compound of the present invention was prepared according to the following prescription.

| | |
|---|---|
| Compound <u>a</u> of the present invention | 0.0001 g |
| Liquid paraffin | 10 g |
| Sterile purified water | suitable amount |
| Total amount | 100 g |

**Example 10**

An eye ointment containing the compound of the present invention was prepared according to the following prescription.

| | |
|---|---|
| Compound <u>b</u> of the present invention | 0.0001 g |
| Liquid paraffin | 10 g |
| Sterile purified water | suitable amount |
| Total amount | 100 g |

**Experimental Example 1: Effects on ocular blood flow of normal eye**

Experimental materials

〈animals used〉

Male Dutch color house rabbits weighing about 2 kg were purchased from Fukusaki Rabbitery Co-operation and used upon confirmation of the absence of abnormality in the eye. The rabbits were bred at temperature 23±3°C and humidity 55±100% on solid feed (Labo R Stock, manufactured by NIHON NOSAN KOGYO K.K., 100 g a day), while allowing free access to tap water.

〈test drugs〉

The inventive compound a was dissolved in physiological saline at a concentration of 0.001 w/v% to give an ophthalmic solution (hereinafter sometimes referred to as 0.001% ophthalmic solution).

1) Measurement of blood flow in optic nerve head

(1) Puncture of different electrode

The rabbits were placed in a retaining cage and optic nerve head was examined for abnormality upon mydriasis with mydrin P (trademark, manufactured by SANTEN PHARMACEUTICAL CO., LTD.). Urethane (dissolved in distilled water at 20%, 1 g/kg) was subcutaneously administered in the abdomen for systemic anesthesia. One or two hours later, a plate type indifferent electrode (manufactured by Biomedical Science, BE-R10) was subcutaneously attached to the head under stable anesthesia The upper and lower eyelids of both eyes were pulled open upward and downward with a suture and conjunctiva at 6 o'clock was incised. The suture was passed through subrectus muscle and pulled downward to fix the eyeball. Sciera at about 3 mm from corneal limbus at 6 o'clock was opened with a 27G needle and a needle type different electrode (manufactured by Biomedical Science, BE-NSP 450-30) was punctured from there through vitreous body into optic nerve head. The opening of the sclera and different electrode were fixed with Aron Alpha (trademark, manufactured by KONISHI CO., LTD.)

(2) Measurement of blood flow

After puncture of different electrode, the rabbits were stood for about one hour. The rabbits were set to inhale 10% hydrogen for about 5 min, and the height from the base line to the curve of the clearance curve depicted on a recorder was measured at 12-second intervals starting from the peak using hydrogen gas clearance tissue flow meter (DHM-3001, manufactured by M.T. Technique). The relationship between the value obtained and the time was plotted on a semilogarithm graph. A straight line was drawn to gather a greatest number possible of measurement dots and half-life ($T_{1/2}$) was calculated from the straight line. The blood flow was determined from the following theoretical formula of Kety (Journal of Clinical Investigate, vol. 27, pp. 476-483 (1948)). The blood flow was measured at 15-min intervals for 90 minutes after instillation and 30-min intervals thereafter till 180 minutes from the instillation. The initial value was an average of two measurements before starting the test.

$$\text{Blood flow (ml/min/100 g)} = 69.3/T_{1/2}$$

(3) Measurement of blood pressure

The auricular artery of house rabbits was secured and pressure transducer was connected thereto to monitor the blood pressure. 2) Instillation method

A 0.001% ophthalmic solution (20 µl) was instilled into one eye and physiological saline (20 µl) was instilled into the other eye.

3) Test

The significant difference test was conducted using a paired t-test, and expressed by the significant difference relative to the control eye.

4) Results

The relative optic nerve head blood flow (blood flow when initial value was 100%) and the relative blood pressure (blood pressure when initial value was 100%) after instillation of the 0.001% ophthalmic solution are shown in Fig. 1 and Fig. 2, respectively. The initial value of the blood flow before instillation was 31.9-79.3 ml/min/100 g and the initial value of the blood pressure before instillation was 73.0 - 92.3 mmHg. The eye after instillation of the 0.001% ophthalmic solution showed a significantly increased blood flow by 4% from 15 mm after the instillation as compared to the initial value, and at 30 mm after instillation, the increase of 17% was obtained as compared to the initial value. The action lasted for 180 min after the instillation. The blood pressure under anesthesia was about 80 mmHg and the effect on the blood pressure after instillation of the 0.001% ophthalmic solution was not found in 180 mm after the instillation.

It is postulated that the 0.001% ophthalmic solution increased the blood flow in the optic nerve head, because the compound of the present invention acted on vascular smooth muscle cell in the optic nerve head via cornea to cause vasodilation, and because the compound of the present invention caused dilation of short posterior ciliary artery via sciera to ultimately increase the retinochoroidal circulation.

**Experimental Example 2** : Effects on ocular blood flow of normal eye

1) Measurement of the blood flow in the optic nerve head

Measured by the same method as in Experimental Example 1.

2) Instillation method

In the same manner as in Experimental Example 1 except that an ophthalmic solution containing the compound b of the present invention dissolved in physiological saline at a concentration of $10^{-4}$w/v% (hereinafter sometimes referred to as $10^{-4}$% ophthalmic solution) was used instead of the 0.001% ophthalmic solution containing the compound a of the present invention used in Experimental Example 1, the ophthalmic solution was instilled.

3) Test

Tested by the same method as in Experimental Example 1.

4) Results

The relative optic nerve head blood flow (blood flow when initial value was 100%) after instillation of the $10^{-4}$% ophthalmic solution is shown in Fig. 3. The initial value of the blood flow before instillation was 41.0 - 85.7 ml/min/100 g.

As compared to the initial value, the eye alter instillation of the $10^{-4}$% ophthalmic solution showed, from 15 min after the instillation, a slight increase in the blood flow from the initial value, and thereafter further increased blood flow significantly, reaching the maximum increase of 23% at 60 min after instillation, which effect lasted for 180 min after the instillation.

It is postulated that the $10^{-4}$% ophthalmic solution increased the blood flow in the optic nerve head because the compound of the present invention acted on vascular smooth muscle cell in the optic nerve head via cornea to cause vasodilation, and because the compound of the present invention caused dilation of short posterior ciliary artery via sciera to ultimately increase the retinochoroidal circulation.

**Experimental Example 3** : Effects on blood flow of ET-1 ocular circulation disorder model

1) Measurement of the blood flow in the optic nerve head

Measured in the same manner as in Experimental Example 1, except that measurement was done immediately before Er-1 injection and at 30-minute intervals after ET-1 injection till 180 minutes after the injection.

2) Instillation method

Using the same ophthalmic solution as in Experimental Example 2 ($10^{-4}$ ophthalmic solution), the solution was instilled according to a similar method.

3) Administration of ET-1

At 60 minutes after the instillation of the $10^{-4}$% ophthalmic solution, $10^{-6}$ M ET-1 (10 μl, derived from human, manufactured by Funakoshi) was injected into the central part of the vitreous body of both eyes while observing the eye ground with a vitrectomy lens.

4) Observation of retinal blood vessel diameter

The retinal blood vessel diameter was macroscopically observed immediately before injection of ET-1 and at 30-minute intervals after ET-1 injection for 180 minutes using an inverted image mirror after measurement of the blood flow according to the following evaluation criteria (score 0-4).

| | |
|---|---|
| Normal retinal bloodvessel diameter | score 0 |
| Contraction of retinal blood vessel dia meter by 1/4 | score 1 |
| Contraction of retinal blood vessel diameter by 1/2 | score 2 |
| Contraction of retinal blood vessel diameter by 3/4 | score 3 |
| Presence of blood vessel barely confirmed or Complete occlusion | score 4 |

5) Test

The blood flow test was performed by the same method as used in Experimental Example 1. However, significant difference from the control eye was expressed in terms of the variation in the blood flow at each time point from that immediately before ET-1 injection. The retinal blood vessel diameter was observed by Wilcoxon test, and expressed by significant difference from control eye.

6) Results

The effect on the decrease in relative optic nerve head blood flow due to ET-1 injection, after instillation of $10^{-4}$% ophthalmic solution, is shown in Fig. 4. The initial value of the blood flow before instillation was 32.4-79.3 ml/min/100 g.

In the control eye, the blood flow at 30 minutes after ET-1 injection reduced by 25% as compared to the blood flow immediately before ET-1 injection. The blood flow further reduced and at 120 minutes after ET-1 injection, it decreased by 46% as compared to the blood flow immediately before ET-1 injection. The decrease lasted till 180 minutes after ET-1 injection.

In the eye instilled with the $10^{-4}$% ophthalmic solution, a decrease in blood flow was suppressed nearly completely from 30 minutes to 90 minutes after ET-1 injection. A decrease in the blood flow by 10% at 120 minutes after ET-1 injection, a decrease in the blood flow by 19% at 150 minutes after ET-1 injection, and a decrease in the blood flow by 20% at 180 minutes after ET-1 injection, all relative to the blood flow immediately before ET-1 injection, were found. However, decrease in the blood flow was significantly suppressed as compared to the control eye. The percent suppression of the $10^{-4}$% ophthalmic solution to the decrease in the blood flow till 180 minutes after the ET-1 injection as determined by time - area under the curve (AUC) was 75%.

The effect against the contraction of retinal blood vessel due to ET-1 injection, after instillation of the $10^{-4}$% ophthalmic solution, is shown in Fig. 5. In the control eye, the retinal blood vessel contracted by 1/2 or above (score 2) from 30 minutes after ET-1 injection, by 3/4 or above (score 3) at 60 minutes after ET-1 injection, and was completely obstructed at 90 minutes after ET-1 injection (score 4). The retinal blood vessel was completely obstructed (score 4) till 180 minutes after ET-1 injection. In contrast, in the eye instilled with $10^{-4}$% ophthalmic solution, the contraction of the retinal blood vessel was significantly suppressed till 90 minutes after ET-1 injection as compared to the control eye. It is postulated that the $10^{-4}$ ophthalmic solution suppressed contraction of the retinal blood vessel and reduction of blood flow in the optic nerve head of the eye with circulation disorder due to ET-1, because the compound of the present invention opened the potassium channel, caused hyperpolarization of cell membrane and suppressed the influx of calcium ion into vascular smooth muscle cells, as well as the release of calcium ion from intracellular sarcoplasmic reticulum.

As is evident from the foregoing explanation, the agent for ameliorating an ocular circulatory disorder of the

present invention increases optic nerve head blood flow of normal eye particularly by instillation, and inhibits vasocontraction of retinal blood vessel and decrease in optic nerve head blood flow both caused by ET-1, without substantial influence on blood pressure. Therefore, the inventive compound is suggested to be effective as a therapeutic agent against glaucoma (particularly normal tension glaucoma caused by an ocular circulation disorder), retinitis pigmentosa, macular degeneration, ischemic optic neuropathy, iridocycitis, retinal artery occlusion, retinal vein occlusion, diabetic retinopathy, ischemic optic neuropathy, choroidal disease following retinal lesion, retinochoroidal disease accompanied by systemic disease, and the like.

This application is based on application No.348738/1996 filed in Japan, the content of which is incorporated hereinto by reference.

## Claims

1. An agent for ameliorating an ocular circulatory disorder comprising a potassium channel opener except cromakalim as an active ingredient.

2. The agent for ameliorating an ocular circulatory disorder of claim 1, wherein the potassium channel opener is a carboxyimidamide derivative of the following formula (I)

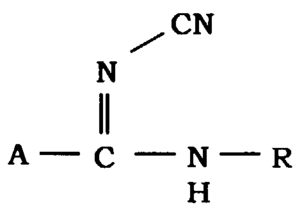

(I)

wherein A is a group of the formula

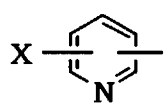

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom,

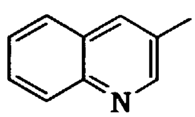 , 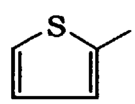 , 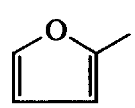 , 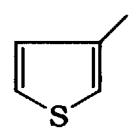 ,

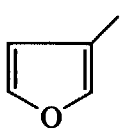 , 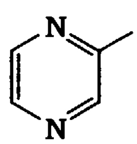 , 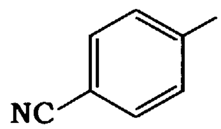

or $H_3C$-S-;
when A is

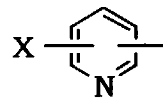

then R should be -$R^1$ a group of the formula

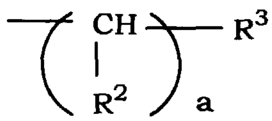

wherein $R^1$ is alkyl, alkyl having nitroxyl, a group of the formula

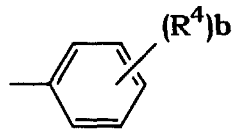

wherein $R^4$ is alkyl or alkoxyl, and b is 0 or 1, or

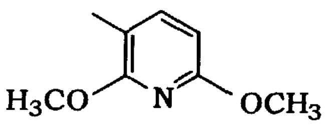

$R^2$ is one or more groups selected from the group consisting of alkyl, aryl, nitroxyl, arylalkoxyl, hydroxyl and hydrogen atom,
a is an integer of 1 to 3, and when a is 2 or above, two or more $R^2$ may be one or more from the groups and the atom of the above-mentioned group, and
$R^3$ is a group of the formula

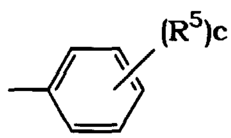

wherein $R^5$ is one or more groups selected from the group consisting of alkyl, alkoxyl, arylalkoxyl, nitro, amino, alkylamino, arylalkylamino, alkylthio, perfluoroalkyl and halogen atom, and c is an integer of 0 to 5, and when c is 2 or above, two or more $R^5$ may be one or more from the groups and the atom of the above-mentioned group,

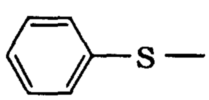

or

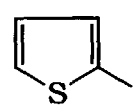

and when A is

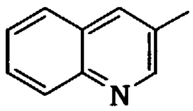 , 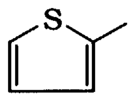 , 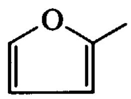 , 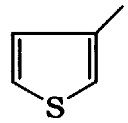 ,

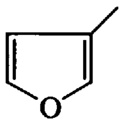 , 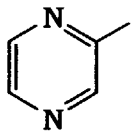 , 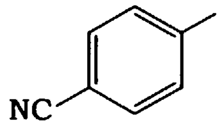

or $H_3C$-S-, then R should be

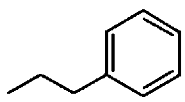 or 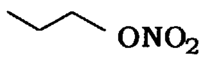

an acid addition salt thereofor a hydrate thereof.

3. The agent for ameliorating an ocular circulatory disorder of claim 2, wherein the carboxyimidamide derivative of the formula (I) is a pyridinecarboxyimidamide derivative of the following formula (II)

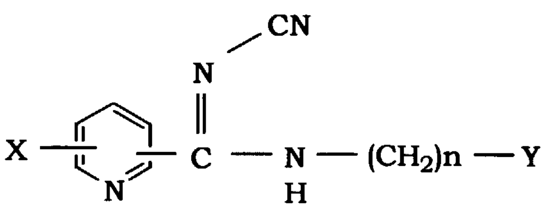 (II)

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom, Y is nitroxyl, methyl, or phenyl optionally substituted by alkyl, alkoxyl, arylalkoxyl, nitro, amino, alkylamino, arylalkylamino, alkylthio, perfluoroalkyl or halogen atom, and n is an integer of 0 to 3.

4. The agent for ameliorating an ocular circulatory disorder of claim 3, wherein the pyridinecarboxyimidamide derivative of the formula (II) is a pyridinecarboxyimidamide derivative of the following formula (III)

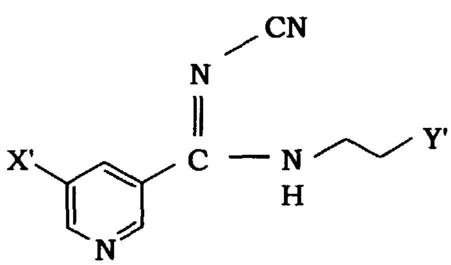 (III)

wherein X' is hydrogen atom or amino, and Y' is nitroxyl or 2-chlorophenyl.

5. The agent for ameliorating an ocular circulatory disorder of claim 4, wherein the pyridinecarboxyimidamide derivative of the formula (III) is N-cyano-N'-(2-nitroxyethyl)-3-pyridinecarboxyimidamide.

6. The agent for ameliorating an ocular circulatory disorder of claim 4, wherein the pyridinecarboxyimidamide derivative of the formula (III) is 5-amino-N-cyano-N'-[2-(2-chlorophenyl)ethyl]-3-pyridinecarboxyimidamide.

7. The agent for ameliorating an ocular circulatory disorder of any of claim 1 to claim 6, which is in the form of an eye drop.

8. The agent for ameliorating an ocular circulatory disorder of any of claim 1 to claim 6, which is in the form of an eye ointment.

9. The agent for ameliorating an ocular circulatory disorder of any of claim 1 to claim 8, which is an agent for the prophylaxis and treatment of a disease caused by a circulatory disorder in the ciliary artery system.

10. The agent for ameliorating an ocular circulatory disorder of claim 9, wherein the disease caused by the circulatory disorder in the ciliary artery system is a disease selected from the group consisting of glaucoma, retinitis pigmentosa, macular degeneration, ischemic optic neuropathy and iridocycitis.

11. The agent for ameliorating an ocular circulatory disorder of claim 9, wherein the disease caused by the circulatory disorder in the ciliary artery system is normal tension glaucoma.

12. The agent for ameliorating an ocular circulatory disorder of claim 4, which is an agent for the prophylaxis or treatment of glaucoma.

13. The agent for ameliorating an ocular circulatory disorder of any of claim 1 to claim 8, which is an agent for the prophylaxis and treatment of a disease caused by a circulatory disorder in the central retinal artery system.

14. The agent for ameliorating an ocular circulatory disorder of claim 13, wherein the disease caused by the circulatory disorder in the central retinal artery system is a disease selected from the group consisting of retinal artery occlusion, retinal vein occlusion, diabetic retinopathy, ischemic optic neuropathy, choroidal disease following retinal lesion and retinochoroidal disease accompanied by systemic disease.

15. A method for ameliorating an ocular circulatory disorder comprising administering a potassium channel opener except cromakalim in an amount effective for amelioration of the an ocular circulatory disorder.

16. The method for ameliorating an ocular circulatory disorder of claim 15, wherein the potassium channel opener is a carboxyimidamide derivative of the following formula (I)

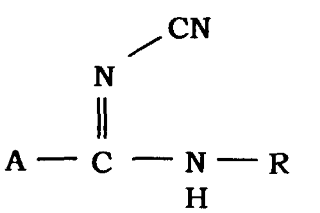

(I)

wherein A is a group of the formula

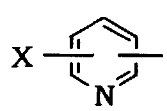

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom,

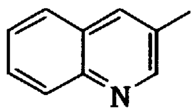 , 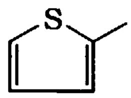 , 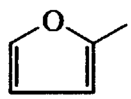 , 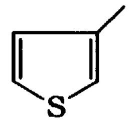 ,

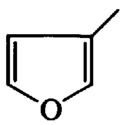 , 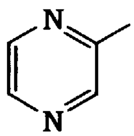 , 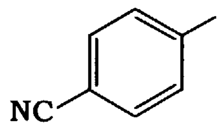

or $H_3C$-S-;
when A is

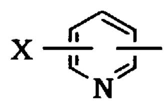

then R should be -$R^1$ a group of the formula

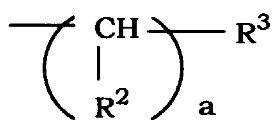

wherein $R^1$ is alkyl, alkyl having nitroxyl, a group of the formula

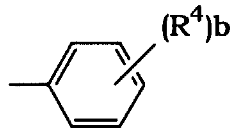

wherein $R^4$ is alkyl or alkoxyl, and b is 0 or 1, or

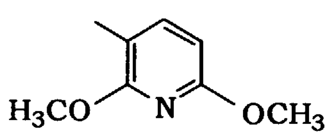

$R^2$ is one or more groups selected from the group consisting of alkyl, aryl, nitroxyl, arylalkoxyl, hydroxyl and hydrogen atom,
a is an integer of 1 to 3, and when a is 2 or above, two or more $R^2$ may be one or more from the groups and the atom of the above-mentioned group, and
$R^3$ is a group of the formula

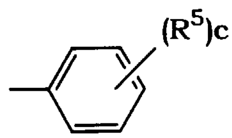

wherein $R^5$ is one or more groups selected from the group consisting of alkyl, alkoxyl, arylalkoxyl, nitro, amino, alkylamino, arylalkylamino, alkylthio, perfluoroalkyl and halogen atom, and c is an integer of 0 to 5, and when c is 2 or above, two or more $R^5$ may be one or more from the groups and the atom of the above-mentioned group,

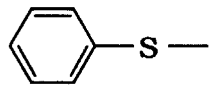 or 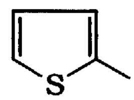

and when A is

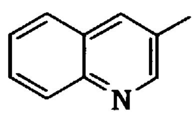 , 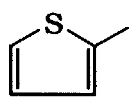 , 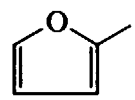 , 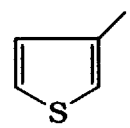 ,

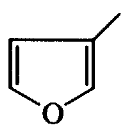 , 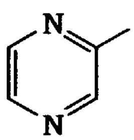 , 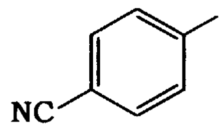

or $H_3C$-S-, then R should be

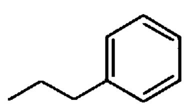 or 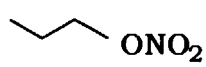

an acid addition salt thereofor a hydrate thereof.

**17.** The method for ameliorating an ocular circulatory disorder of claim 16, wherein the carboxyimidamide derivative of the formula (I) is a pyridinecarboxyimidamide derivative of the following formula (II)

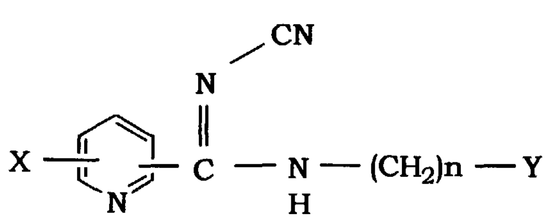

(II)

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom, Y is nitroxyl, methyl, or phenyl optionally

substituted by alkyl, alkoxyl, arylalkoxyl, nitro, amino, alkylamino, arylalkylamino, alkylthio, perfluoroalkyl or halogen atom, and n is an integer of 0 to 3.

**18.** The method for ameliorating an ocular circulatory disorder of claim 17, wherein the pyridinecarboxyimidamide derivative of the formula (II) is a pyridinecarboxyimidamide derivative of the following formula (III)

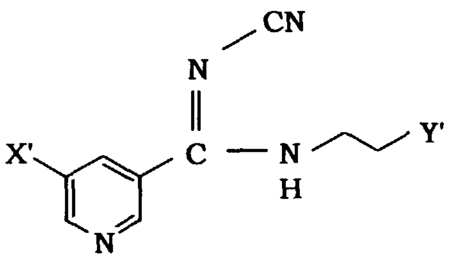

(III)

wherein X' is hydrogen atom or amino, and Y' is nitroxyl or 2-chlorophenyl.

**19.** The method for ameliorating an ocular circulatory disorder of claim 18, wherein the pyridinecarboxyimidamide derivative of the formula (III) is N-cyano-N'-(2-nitroxyethyl)-3-pyridinecarboxyimidamide.

**20.** The method for ameliorating an ocular circulatory disorder of claim 18, wherein the pyridinecarboxyimidamide derivative of the formula (III) is 5-amino-N-cyano-N'-[2-(2-chlorophenyl)ethyl]-3-pyridinecarboxyimidamide.

**21.** The method for ameliorating an ocular circulatory disorder of any of claim 15 to claim 20, wherein the calcium channel opener is administered in the form of an eye drop.

**22.** The method for ameliorating an ocular circulatory disorder of any of claim 15 to claim 20, wherein the calcium channel opener is administered in the form of an eye ointment.

**23.** The method for ameliorating an ocular circulatory disorder of any of claim 15 to claim 22, which is a method for the prophylaxis and treatment of a disease caused by a circulatory disorder in the ciliary artery system.

**24.** The method for ameliorating an ocular circulatory disorder of claim 23, wherein the disease caused by the circulatory disorder in the ciliary artery system is a disease selected from the group consisting of glaucoma, retinitis pigmentosa, macular degeneration, ischemic optic neuropathy and iridocycitis.

**25.** The method for ameliorating an ocular circulatory disorder of claim 23, wherein the disease caused by the circulatory disorder in the ciliaxy artery system is normal tension glaucoma

**26.** The method for ameliorating an ocular circulatory disorder of claim 18, which is a method for the prophylaxis or treatment of glaucoma

**27.** The method for ameliorating an ocular circulatory disorder of any of claim 15 to claim 22, which is an method for the prophylaxis and treatment of a disease caused by a circulatory disorder in the central retinal artery system.

**28.** The method for ameliorating an ocular circulatory disorder of claim 27, wherein the disease caused by the circulatory disorder in the central retinal artery system is a disease selected from the group consisting of retinal artery occlusion, retinal vein occlusion, diabetic retinopathy, ischemic optic neuropathy, choroidal disease following retinal lesion and retinochoroidal disease accompanied by systemic disease.

**29.** Use of a potassium channel opener except cromakalim for the production of an agent for ameliorating an ocular circulatory disorder.

**30.** The use of claim 29, wherein the potassium channel opener is a carboxyimidamide derivative of the following formula (I)

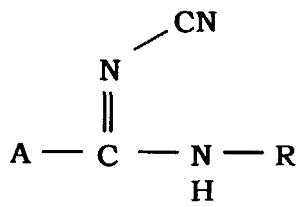 (I)

wherein A is a group of the formula

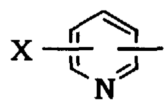

wherein X is alkyl, hydroxyalkyl, carboxyl, amino, acylamino, alkylamino, dialkylamino, axylalkylamino, alkylsulfonamido, bisalkylsulfonylamino, hydroxyl, halogen atom or hydrogen atom,

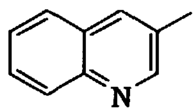 , 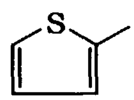 , 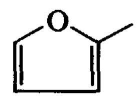 , 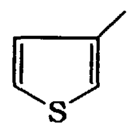 ,

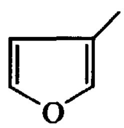 , 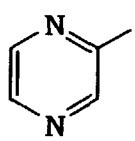 , 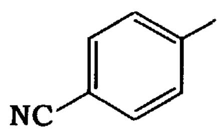

or $H_3C$-S-;
when A is

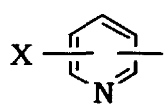

then R should be $-R^1$ a group of the formula

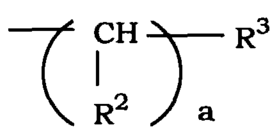

wherein $R^1$ is alkyl, alkyl having nitroxyl, a group of the formula

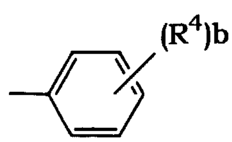

wherein $R^4$ is alkyl or alkoxyl, and b is 0 or 1, or

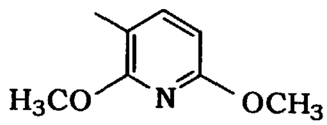

$R^2$ is one or more groups selected from the group consisting of alkyl, aryl, nitroxyl, arylalkoxyl, hydroxyl and hydrogen atom,
a is an integer of 1 to 3, and when a is 2 or above, two or more $R^2$ may be one or more from the groups and the atom of the above-mentioned group, and
$R^3$ is a group of the formula

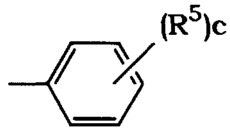

wherein $R^5$ is one or more groups selected from the group consisting of alkyl, alkoxyl, arylalkoxyl, nitro, amino, alkylamino, arylalkylamino, alkylthio, perfluoroalkyl and halogen atom, and c is an integer of 0 to 5, and when c is 2 or above, two or more $R^5$ may be one or more from the groups and the atom of the above-mentioned group,

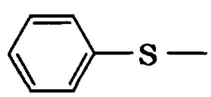 or 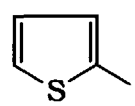

and when A is

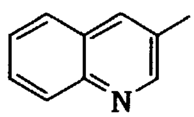, 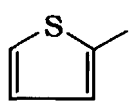, 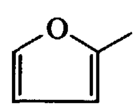, 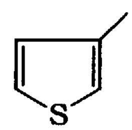,

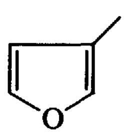, 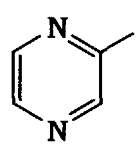, 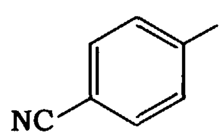

or $H_3C$-S-, then R should be

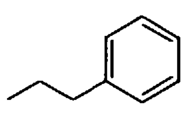 or 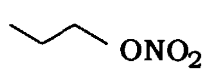

an acid addition salt thereofor a hydrate thereof.

**31.** The use of claim 30, wherein the carboxyimidamide derivative of the formula (I) is a pyridinecarboxyimidamide derivative of the following formula (II)

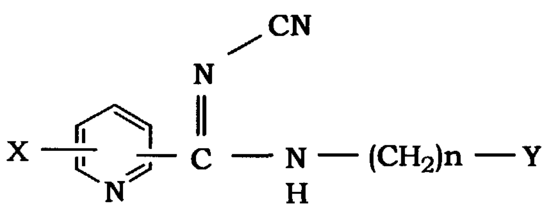

(II)

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom, Y is nitroxyl, methyl, or phenyl optionally substituted by alkyl, alkoxyl, arylalkoxyl, nitro, amino, alkylamino, arylalkylamino, alkylthio, perfluoroalkyl or halogen atom, and n is an integer of 0 to 3.

**32.** The use of claim 31, wherein the pyridinecarboxyimidamide derivative of the formula (II) is a pyridinecarboxyimidamide derivative of the following formula (III)

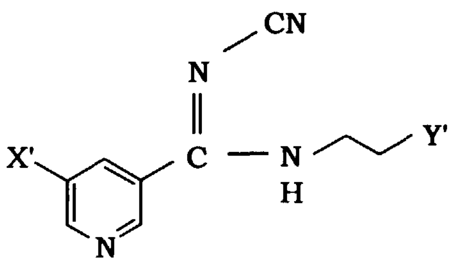

(III)

wherein X' is hydrogen atom or amino, and Y' is nitroxyl or 2-chlorophenyl.

**33.** The use of claim 32, wherein the pyridinecarboxyimidamide derivative of the formula (III) is N-cyano-N'-(2-nitroxyethyl)-3-pyridinecarboxyimidamide.

**34.** The use of claim 32, wherein the pyridinecarboxyimidamide derivative of the formula (III) is 5-amino-N-cyano-N'-[2-(2-chlorophenyl)ethyl]-3-pyridinecarboxyimidamide.

**35.** The use of any of claim 29 to claim 34, wherein the agent is in the form of an eye drop.

**36.** The use of any of claim 29 to claim 34, wherein the agent is in the form of an eye ointment.

**37.** The use of any of claim 29 to claim 36, wherein the agent is for the prophylaxis and treatment of a disease caused by a circulatory disorder in the ciliary artery system.

**38.** The use of claim 37, wherein the disease caused by the circulatory disorder in the ciliary artery system is a disease selected from the group consisting of glaucoma, retinitis pigmentosa, macular degeneration, ischemic optic neuropathy and iridocyclitis.

**39.** The use of claim 37, wherein the disease caused by the circulatory disorder in the ciliary artery system is normal tension glaucoma.

**40.** The use of claim 32, wherein the agent is for the prophylaxis or treatment of glaucoma.

**41.** The use of any of claim 29 to claim 36, wherein the agent is for the prophylaxis and treatment of a disease caused by a circulatory disorder in the central retinal artery system.

**42.** The use of claim 41, wherein the disease caused by the circulatory disorder in the central retinal artery system is

a disease selected from the group consisting of retinal artery occlusion, retinal vein occlusion, diabetic retinopathy, ischemic optic neuropathy, choroidal disease following retinal lesion and retinochoroidal disease accompanied by systemic disease.

**43.** A pharmaceutical composition for ameliorating an ocular circulatory disorder, comprising an effective amount of a potassium channel opener except cromakalim and a pharmaceutically acceptable carrier.

**44.** The pharmaceutical composition of claim 43, wherein the potassium channel opener is a carboxyimidamide derivative of the following formula (I)

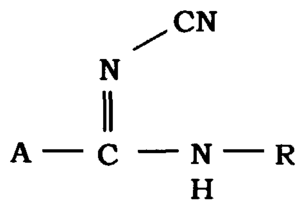

(I)

wherein A is a group of the formula

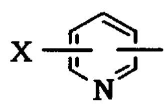

wherein X is alkyl, hydroxyalkyl, carboxyl, amino, acylamino, alkylamino, dialkylamino, axylalkylamino, alkylsulfonamido, bisalkylsulfonylamino, hydroxyl, halogen atom or hydrogen atom,

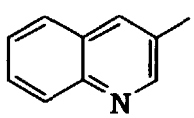 , 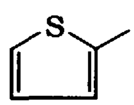 , 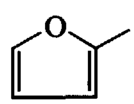 , 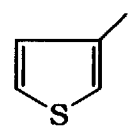 ,

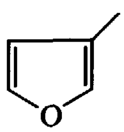 , 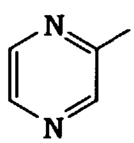 , 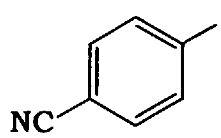

or $H_3C$-S-;
when A is

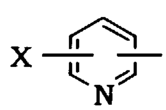

then R should be -$R^1$ or a group of the formula

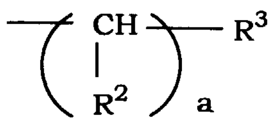

wherein $R^1$ is alkyl, alkyl having nitroxyl, a group of the formula

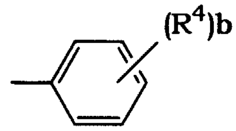

wherein $R^4$ is alkyl or alkoxyl, and b is 0 or 1, or

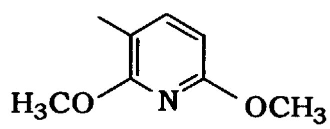

$R^2$ is one or more groups selected from the group consisting of alkyl, aryl, nitroxyl, arylalkoxyl, hydroxyl and hydrogen atom,
a is an integer of 1 to 3, and when a is 2 or above, two or more $R^2$ may be one or more from the groups and the atom of the above-mentioned group, and
$R^3$ is a group of the formula

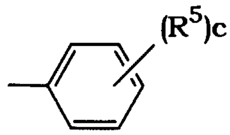

wherein $R^5$ is one or more groups selected from the group consisting of alkyl, alkoxyl, arylalkoxyl, nitro, amino, alkylamino, arylalkylamino, alkylthio, perfluoroalkyl and halogen atom, and c is an integer of 0 to 5, and when c is 2 or above, two or more $R^5$ may be one or more from the groups and the atom of the above-mentioned group,

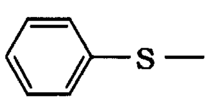

or

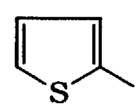

and when A is

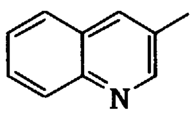, 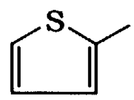, 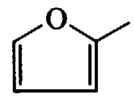, 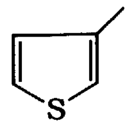,

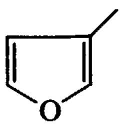, 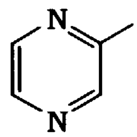, 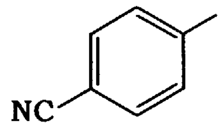

or $H_3C$-S-, then R should be

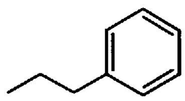 or 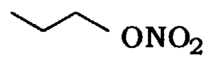

an acid addition salt thereof or a hydrate thereof.

**45.** The pharmaceutical composition of claim 44, wherein the carboxyimidamide derivative of the formula (I) is a pyridinecarboxyimidamide derivative of the following formula (II)

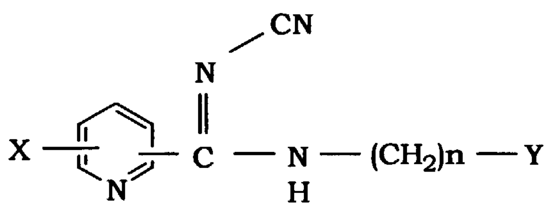

(II)

wherein X is alkyl, hydroxyalkyl, carboxyl, amino, acylamino, alkylamino, dialkylamino, arylalkylamino, alkylsulfonamido, bisalkylsulfonylamino, hydroxyl, halogen atom or hydrogen atom, Y is nitroxyl, methyl, or phenyl optionally substituted by alkyl, alkoxyl, arylalkoxyl, nitro, amino, alkylamino, arylalkylamino, alkylthio, perfluoroalkyl or halogen atom, and n is an integer of 0 to 3.

**46.** The pharmaceutical composition of claim 45, wherein the pyridinecarboxyimidamide derivative of the formula (II) is a pyridinecarboxyimidamide derivative of the following formula (III)

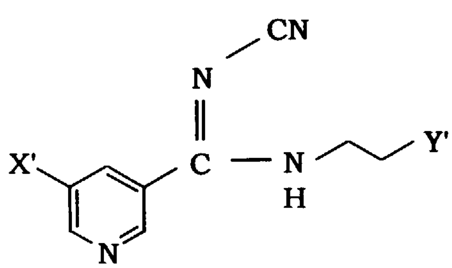

(III)

wherein X' is hydrogen atom or amino, and Y' is nitroxyl or 2-chlorophenyl.

**47.** The pharmaceutical composition of claim 46 wherein the pyridinecarboxyimidamide derivative of the formula (III) is N-cyano-N'-(2-nitroxyethyl)-3-pyridinecarboxyimidamide.

**48.** The pharmaceutical composition of claim 46, wherein the pyridinecarboxyimidamide derivative of the formula (III) is 5-amino-N-cyano-N'-[2-(2-chlorophenyl)ethyl]-3-pyridinecarboxyimidamide.

**49.** The pharmaceutical composition of any of claim 43 to claim 48, which is in the form of an eye drop.

**50.** The pharmaceutical composition of any of claim 43 to claim 48, which is in the form of an eye ointment.

**51.** The pharmaceutical composition of any of claim 43 to claim 50, which is a composition for the prophylaxis and treatment of a disease caused by a circulatory disorder in the ciliary artery system.

**52.** The pharmaceutical composition of claim 51, wherein the disease caused by the circulatory disorder in the ciliary artery system is a disease selected from the group consisting of glaucoma, retinitis pigmentosa, macular degeneration, ischemic optic neuropathy and iridocyditis.

**53.** The pharmaceutical composition of claim 51, wherein the disease caused by the circulatory disorder in the ciliary artery system is normal tension glaucoma

**54.** The pharmaceutical composition of claim 46, which is a composition for the prophylaxis or treatment of glaucoma

**55.** The pharmaceutical composition of any of claim 43 to claim 50, which is a composition for the prophylaxis and treatment of a disease caused by a circulatory disorder in the central retinal artery system.

**56.** The pharmaceutical composition of claim 55, wherein the disease caused by the circulatory disorder in the central retinal artery system is a disease selected from the group consisting of retinal artery occlusion, retinal vein occlusion, diabetic retinopathy, ischemic optic neuropathy, choroidal disease following retinal lesion and retinochoroidal disease accompanied by systemic disease.

**57.** A commercial package comprising the pharmaceutical composition of any of claim 43 to claim 56 and a written matter associated therewith, the written matter stating that the pharmaceutical composition can or should be used for ameliorating an ocular circulatory disorder.

FIG. 1

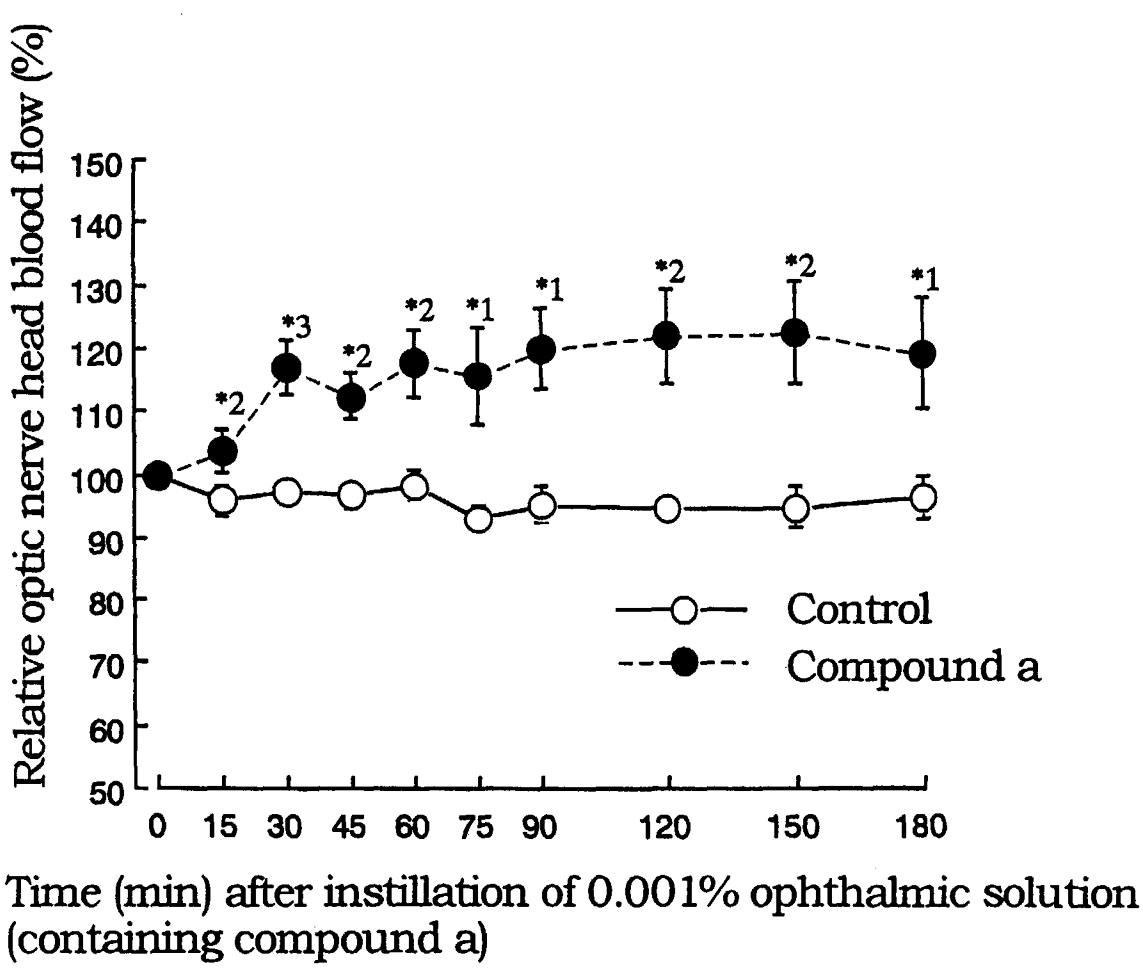
Relative optic nerve head blood flow (%)
150
140
130
120
110
100
90
80
70
60
50
0 15 30 45 60 75 90 120 150 180
*2
*3
*2
*2
*1
*1
*2
*2
*1
Control
Compound a
Time (min) after instillation of 0.001% ophthalmic solution (containing compound a)

FIG. 2

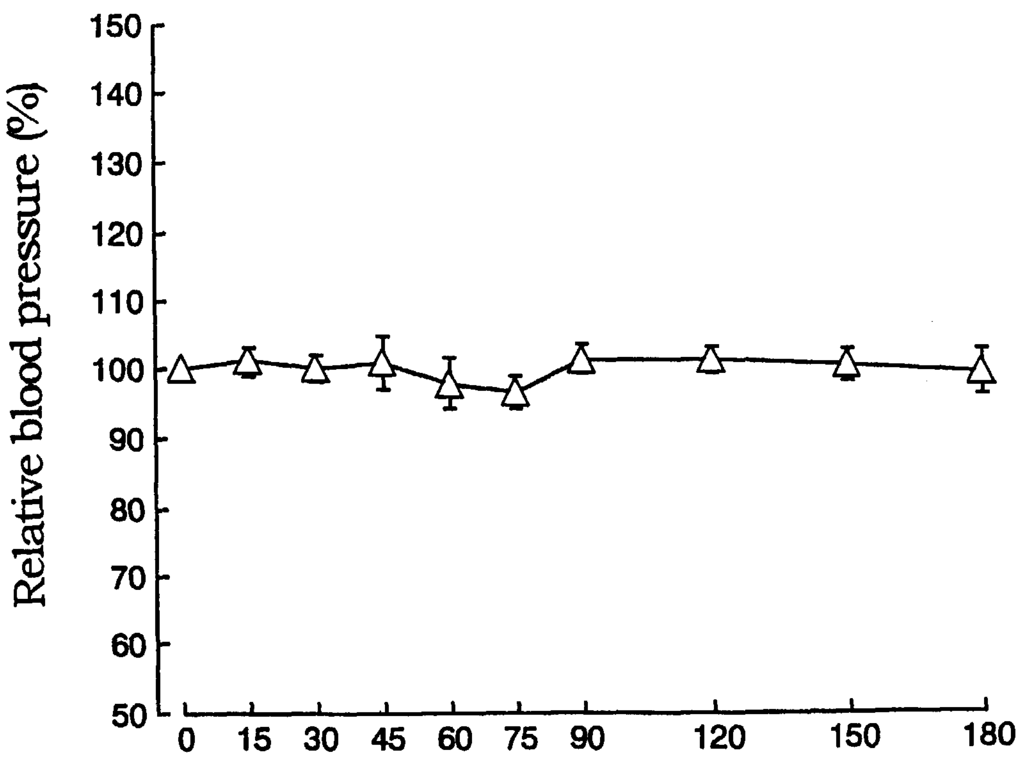


Time (min) after instillation of 0.001% ophthalmic solution (containing compound a)

FIG. 3

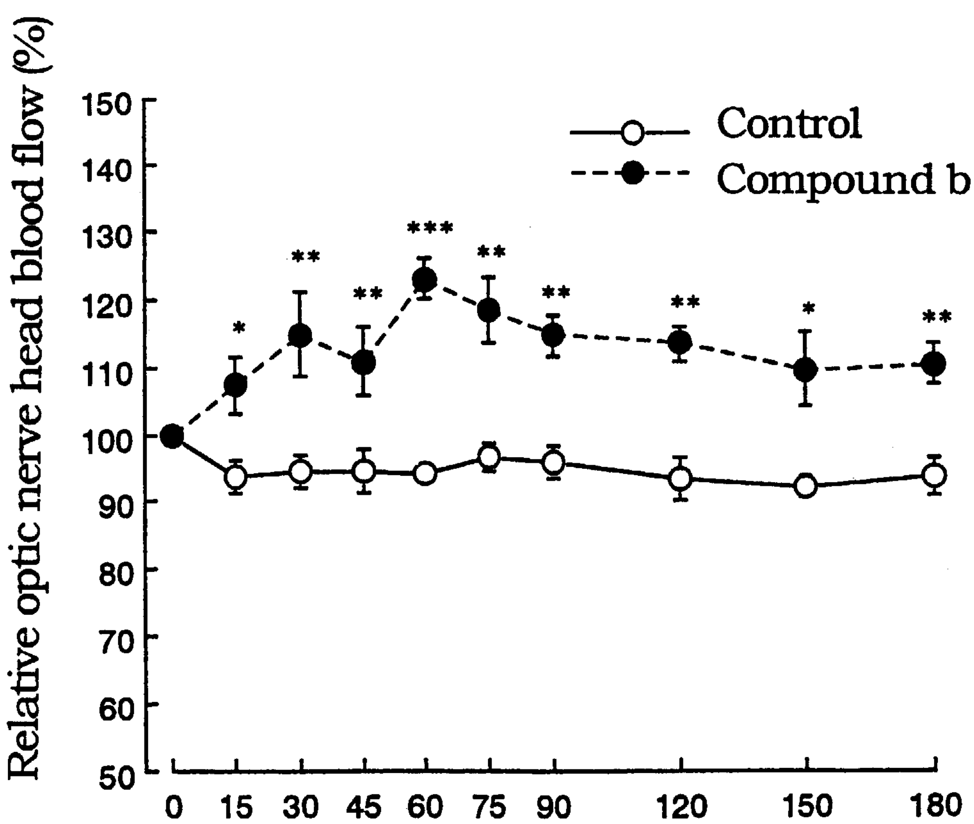


Time (min) after instillation of $10^{-4}$ % ophthalmic solution (containing compound b)

FIG. 4

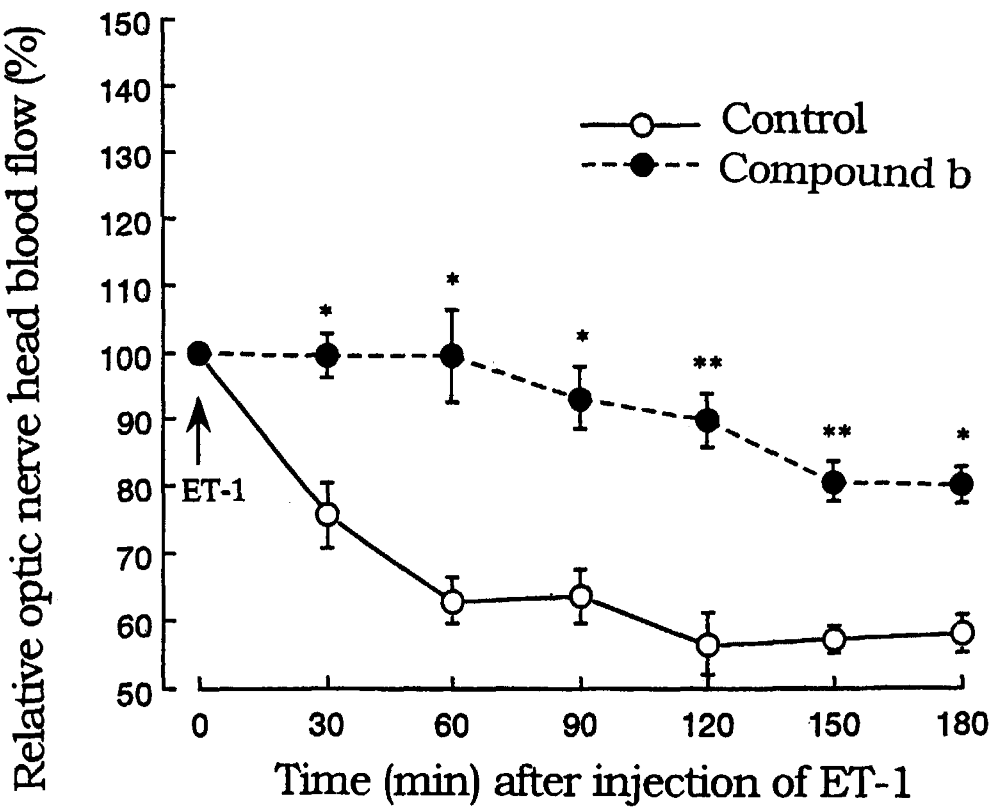
Relative optic nerve head blood flow (%)
150
140
130
120
110
100
90
80
70
60
50
Control
Compound b
ET-1
*
*
*
**
**
*
0
30
60
90
120
150
180
Time (min) after injection of ET-1

FIG. 5

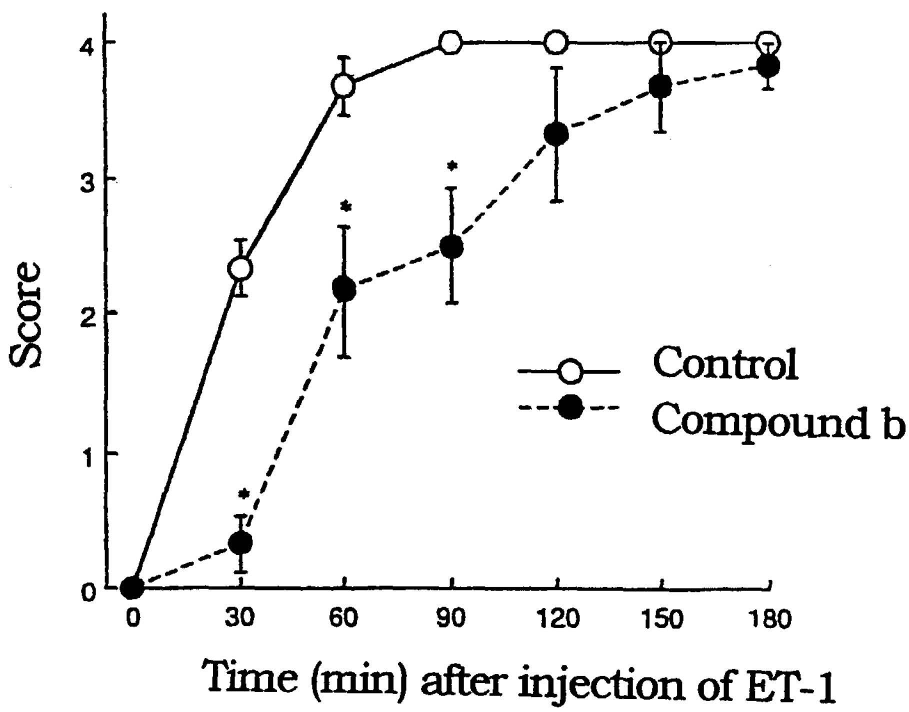
Score
4
3
2
1
0
0
30
60
90
120
150
180
Time (min) after injection of ET-1
Control
Compound b
*

## INTERNATIONAL SEARCH REPORT

International application No.
PCT/JP97/04665

A. CLASSIFICATION OF SUBJECT MATTER
Int.Cl[6] A61K45/00, A61K31/47, A61K31/44, A61K31/50, A61K31/38, A61K31/275

According to International Patent Classification (IPC) or to both national classification and IPC

B. FIELDS SEARCHED

Minimum documentation searched (classification system followed by classification symbols)
Int.Cl[6] A61K45/00, A61K31/47, A61K31/44, A61K31/50, A61K31/38, A61K31/275

Documentation searched other than minimum documentation to the extent that such documents are included in the fields searched

Electronic data base consulted during the international search (name of data base and, where practicable, search terms used)
CA (STN), REGISTRY (STN), WPIDS (STN)

C. DOCUMENTS CONSIDERED TO BE RELEVANT

| Category* | Citation of document, with indication, where appropriate, of the relevant passages | Relevant to claim No. |
|---|---|---|
| X | JP, 2-235868, A (Eli Lilly and Co.), September 18, 1990 (18. 09. 90), Page 4, upper right column, 4th line from the bottom to lower left column, line 5 | 1, 7-12, 29, 35-40, 43, 49-54, 57 |
| A | Reference as a whole | 2-6, 13, 14, 30-34, 41, 42, 44-48, 55, 56 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| March 18, 1998 (18. 03. 98) | March 31, 1998 (31. 03. 98) |
| Name and mailing address of the ISA/ Japanese Patent Office | Authorized officer |
| Facsimile No. | Telephone No. |

Form PCT/ISA/210 (second sheet) (July 1992)